Europäisches Patentamt

European Patent Office

Office européen des brevets

(11) Publication number: **0 097 810**
**A1**

(12) # EUROPEAN PATENT APPLICATION

(21) Application number: 83105050.5

(22) Date of filing: 21.05.83

(51) Int. Cl.³: **A 61 K 7/08**

(30) Priority: 09.06.82 IT 2179082

(43) Date of publication of application:
11.01.84 Bulletin 84/2

(84) Designated Contracting States:
AT BE CH DE FR GB LI LU NL SE

(71) Applicant: RAVIZZA S.p.A.
35, Via Europa
I-20053 Muggio' (Milano)(IT)

(72) Inventor: Verga, Alberto
Via T. da Cazzaniga 9/6
Milan(IT)

(72) Inventor: Sperandeo, Felice
Via Balzac, 3
Milan(IT)

(72) Inventor: Benedetto, Giuseppe
Via Abba 3
Muggio' (Milan)(IT)

(74) Representative: Gervasi, Gemma
Studio Brevetti e Marchi NOTARBARTOLO & GERVASI
33, Viale Bianca Maria
I-20122 Milano(IT)

(54) Enzyme-based shampoo.

(57) Shampoo in liquid or semi-solid anhydrous form, having enzymatic activity which is stable with time, and comprising as its essential components a non-ionic detergent surface active agent chosen from esters of fatty acids with poly-alcohols, either oxyethylenated or not, fatty acids and oxy-ethylenated alkylphenols, and a lipase of pancreatic, vege-table or fungal type.

EP 0 097 810 A1

Croydon Printing Company Ltd.

- 1 -

ENZYME-BASED SHAMPOO

The importance of perfect and balanced cellular, glandular
and vascular functioning of the scalp is well known for the
growth and maintenance of abundant, well distributed hair.

In this respect, the sebaceous gland cells perform a basic
function. On disintegrating in their central part, they
transfer their constituent fats into the pilo-sebaceous duct,
so lubricating the hair and the cutaneous surface.

When the sebaceous and sudoriferous glands function abnormally,
they produce a greater number of fatty substances which make
the scalp, and consequently the hair disposed thereon, bright
and greasy.

In this case it is important to maintain continuous deep
cleaning, prevent degeneration of the already secreted sebum,
and prevent irritation and bad odours arising, in order to
prevent the hair from growing thin and weak on what is already
a difficult surface, with the result that the individual hairs
split easily and easily separate from the follicle under the
smallest tension.

The lipids which represent the fatty substances of the hair and
scalp mostly consist of various fatty acids bonded to glycerin,
but also consist partly of an entire series of alcohols which
give them characteristics similar to waxes.

The average composition of such lipids is given hereinafter (M. Gloor - Determination and analysis of sebum on skin and hairs, Cosmetic Science, Volume I, 225 - Academic Press - 1978):

|  | % |
|---|---|
| Waxy substances: | |
| - triglycerides | 34.65 |
| - free fatty acids | 20.89 |
| - cholesterol waxes and esters | 19.02 |
| - free cholesterol | 8.72 |
| Liquids: | |
| - squalene | 10.96 |
| - paraffins | 5.75 |

These compounds are difficult to eliminate because of their structure.

During normal functioning of the hair system, the bonded fatty substances are split by the effect of other substances present in the organism, and thus also in the pilo-sebaceous ducts, i.e. lipolytic enzymes or lipase. Lipase splits the fats into alcohols and fatty acids, which are more easily removed from the hair by normal washing, the former, i.e. the alcohols, because they are soluble in water, and the latter, i.e. the fats, because they easily saponify with the bases present on the tissues (sodium, potassium, ammonium ions) as normal constituents or as a product of their metabolism.

However, in many cases these endogenous lipases are either in an insufficient quantity and therefore do not solve the problem, or are in excess and lead to other drawbacks (excess of free fatty acids which can give rise to irritation).

In general, in removing fats bonded into complex molecules, it is necessary to use preparations of energetic action, which are poorly soluble and are also aggressive towards the other constituent substances of the hair structure. It is therefore important to carry out a cleaning process which follows the natural physiological mechanisms by introducing into the shampoo an enzyme having a demolishing action on sebaceous fats. From this aspect, it seemed interesting to use lipase.

However, lipase as in the case of all enzymes possesses very precise and rather restricted stability limits. It is sensitive to heat action, to moisture and to a large number of chemical agents, and acts within very narrow pH limits.

For all these reasons, and the resultant difficulty of preserving the final product, the use of enzymes and in particular lipase has been relegated up to the present time to improvised preparations made up by qualified personnel when required.

The object of the present invention is to provide a shampoo containing lipase, in which none of the other ingredients cause degradation of the lipase, and thus enable its enzymatic activity to be preserved unaltered with time. A product of reliable action is therefore provided containing substances of perfect tolerability able to exert a specific action in those cases in which it is necessary to remove the excess sebum from the hair without the use of energetic treatment.

The critical elements which enable the lipase-based shampoo to be prepared, and on which the new formulation according to the invention is therefore based are as follows:
1) absence of water
2) use of non-ionic surface active agents
3) detergent action exerted without foam formation.

With regard to the absence of water, it is already known that this latter has a negative effect on the stability of lipase. From the vast amount of experimental work carried out, it has been found that no aqueous composition containing lipase has a stability in terms of this latter which exceeds seven days at ambient temperature or fifteen days at $4^{\circ}C$.

With regard to the requirement of no foam formation, it has been found that when the air phase breaks the liquid-enzyme interface, there is a considerable reduction in enzymatic activity. This factor has therefore been considered very important in formulating the product.

With regard to the choice of the surface-active agent, this is of fundamental importance in that it constitutes a critical aspect of the formulation according to the present invention, as it has to satisfy the stated requirements, i.e. absence of water in the formulation and absence of foaming effect by the surface-active agent.

The various classes of surface-active agents normally used in the hair cleaning field were tried, namely anionic, amphoteric, cationic and non-ionic.

The use of surface-active agents is essential for hair cleaning. In this respect, substances of high surface-active power disperse in the aqueous solution used, to form micelles which embrace the particles present, whether these are of cutaneous production or of exogenous origin. These micelles therefore enable all undesirable components to be separated from the scalp and from the stem of the hair, these components being made more easily removable by enzymatic splitting.

Nearly all products tested were found unusable for the purpose of the present invention, in that they are available only in aqueous solution either to facilitate their use or because of synthesis requirements.

If however anhydrous products are used, it is found that the presence of anionic surface-active agents, either alone or associated with other classes of surface-active agents leads to a reduction in lipase activity.

Likewise, cationic compounds have been found to be poisons for the category of enzymes tested, because of their activity as modifiers of the reactivity of the enzyme protein components.

Amphoteric components were found to be suitable for association with enzymes where the pH of the medium was close to neutrality.  However, these surface-active agents result in very compact and lasting foam, and thus during use they inhibit enzymatic activity because of the foam produced.

It has been found, and represents a subject matter of the present invention, that there exists a limited group of non-ionic surface active agents which act as dispersants for lipolytic enzymes, do not produce appreciable foam during use, and do not alter enzymatic activity with time, thus possessing all the requirements necessary for preparing the enzymatic shampoo according to the present invention.

More specifically, the following non-ionic surface active agents, free from peroxides, have proved suitable for the formulations according to the invention:
- sorbitan fatty acid esters, either oxyethylenated or not;
- glucose fatty acid esters, either oxyethylenated or not;
- saccharose fatty acid esters, either oxyethylenated or not;
- pentaerythritol fatty acid esters, either oxyethylenated or not;
- glycerol fatty acid esters, either oxyethylenated or not;

- derivatives of $C_8$ to $C_{22}$ chain alcohols, including branched
  alcohols with the saturated and unsaturated bonds oxyethylenated;
- oxyethylenated alkylphenols;
- oxyethylenated derivatives of lanolin alcohols;
- oxyethylenated derivatives of lanolin esters;
- oxyethylenated derivatives of beeswax.

Of the aforesaid series of products, the following have been
found preferable:
- oxyethylenated sorbitan fatty acid esters;
- saccharose fatty acid esters;
- oxyethylenated glycerol fatty acid esters;
- oxyethylenated derivatives of saturated and unsaturated
  $C_{16}$-$C_{18}$ chain alcohols;
- oxyethylenated alkylphenols;
- oxyethylenated derivatives of lanolin alcohols.

The shampoo according to the present invention is therefore
constituted by a formulation in which the following are present
as essential constituents:
a) one or more non-ionic surface active agents chosen from those
   listed heretofore as suitable;
b) a lipase, which can be of pancreatic, vegetable or fungal
   type.

The concentration of the components under point a) is mainly
related to appearance requirements. The minimum quantity
necessary is 8-10%.

The lipase concentration can also vary between wide limits.
The minimum necessary quantity is estimated at 5000 Int.
units per 100 grams of product.

The formulation, which can be in liquid or semi-solid form,
can comprise other ingredients such as perfumes, diluents, excipients and the like, which do not interfere with the two stated essential constituents, and thus must not contain water.

They can also serve to give the product the required consistency.

The lipase preferred in the formulations according to the invention is fungal lipase because of its high activity and versatility.

Pancreatic lipase obtained from slaughtered animals is less advisable. Because of its origin, the possibility of allergic cutaneous reactions cannot be excluded.

Vegetable lipase, obtained from wheat germ, is better from - the tolerability aspect, but its activity is weak and it is very costly.

Formulation examples of shampoos according to the invention are given hereinafter for illustrative purposes only.

EXAMPLE 1

Formulation of a foamless shampoo in the form of a cream, the composition being per 100 grams:

1) Fungal lipase (50,000 Int. units)           0.1 - 2 g
2) Oxyethylenated coconut fatty acid
   mono-diglycerides (40 moles)                      35 g
3) Polyoxyethylenated sorbitanmonolaurate-20         25 g
4) Oxyethylenated nonylphenol (6 moles)              18 g
5) Diethyleneglycol monoethylether                 12.3 g
6) Oxyethylenated oleic alcohol (20 moles)            9 g
7) Perfume                                          0.2 g.

Method of manufacture:

Components 2, 3, 4, 5 and 6 are melted together, the mass is cooled to 37°C, the fungal lipase is added, then the perfume, and cooling is completed to ambient temperature.

Method of use:

Wet the hair abundantly with water, apply the product at several points, massage carefully for a few minutes. Rinse and reapply the product in the same manner as before. The massaging enables the product to more easily reach the sebaceous deposits on the piliferous follicle, and to remove them. The time for which the product remains must be extended to about 15 minutes, which is sufficient to trigger the enzymatic hydrolysis. Finally wash, and dry normally.

EXAMPLE 2

Shampoo for greasy hair with dandruff:

Quantity per 100 g:

1) Fungal lipase (50,000 Int. units)                           0.1 - 2 g
2) Undecylenic monoethanolamide                                     1 g
3) Sulphur dissolved in polyoxyethylenated
   sorbitanmonooleate-20                                           2.5g
4) Saccharose mono-dilaurate                                       25 g
5) Oxyethylenated coconut fatty acid monoglycerides
   (38 moles)                                                      15 g
6) Polyoxyethylene derivatives of coconut fatty acids
   (20 moles)                                                      18 g
7) Oxyethylenated dodecylphenol (10 moles)                         17 g
8) Dipropylene glycol                                             20.5g
9) Perfume                                                   as required.

Melt components 2 to 8 together until completely dissolved. Cool the mass under stirring to 37°C and add the fungal lipase. Perfume. Homogenise the product. Cool to ambient temperature.

- 1 -

0097810

PATENT CLAIMS

1. An enzyme-based shampoo stable with time, consisting of an anhydrous mixture comprising essentially:

a) one or more non-ionic surface active agents which are free from peroxides, are dispersants for lipolytic enzymes, are non-foaming, and possess detergent activity;

b) lipase.

2. A shampoo as claimed in claim 1, wherein the non-ionic surface active agent is present at least in a quantity of 8-10%.

3. A shampoo as claimed in claim 1, wherein the lipase is present at least in a quantity equivalent to 5000 Int. units per 100 grams of product.

4. A shampoo as claimed in claim 1, wherein the non-ionic surface active agents are chosen from the following group:

- sorbitan fatty acid esters, either oxyethylenated or not;
- glucose fatty acid esters, either oxyethylenated or not;
- saccharose fatty acid esters, either oxyethylenated or not;
- pentaerythritol fatty acid esters, either oxyethylenated or not;
- glycerol fatty acid esters, either oxyethylenated or not;
- oxyethylenated $C_8$ to $C_{22}$ linear or branched chain saturated or unsaturated aliphatic alcohols;
- oxyethylenated alkylphenols;
- oxyethylenated derivatives of lanolin alcohols;
- oxyethylenated derivatives of lanolin esters;
- oxyethylenated derivatives of beeswax.

5. A shampoo as claimed in claim 1, wherein the lipase is of pancreatic, vegetable or fungal type.

6. A shampoo as claimed in claim 4, wherein the non-ionic surface active agent is chosen from the following group:
- oxyethylenated sorbitan fatty acid esters;
- saccharose fatty acid esters;
- oxyethylenated glycerol fatty acid esters;
- oxyethylenated $C_{16}$ to $C_{18}$ linear or branched chain saturated or unsaturated aliphatic alcohols;
- oxyethylenated alkylphenols;
- oxyethylenated derivatives of lanolin alcohols.

7. A shampoo as claimed in claim 1 or 5, wherein the lipase is of fungal type.

8. A shampoo as claimed in claim 1, containing the following ingredients in 100 g of product:
- fungal lipase (50,000 Int. units)                     0.1 - 2 g
- oxyethylenated coconut fatty acid
  monoglycerides                                         35 g
- polyoxyethylenated sorbitanmonolaurate-20             25 g
- oxyethylenated nonylphenol                            18 g
- diethyleneglycol monoethylether                       12.3 g
- oxyethylenated oleic alcohol                          9 g
- perfume                                               as required.

9. A shampoo as claimed in claim 1, containing the following ingredients in 100 g of product:
- fungal lipase (50,000 Int. units)                     0.1 - 2 g
- undecylenic monoethanolamide                          1 g
- sulphur dissolved in polyoxyethylenated
  sorbitanmonooleate-20                                 2.5 g
- saccharose mono-dilaurate                             25 g
- oxyethylenated coconut fatty acid
  monoglycerides                                        15 g
- polyoxyethylene derivatives of coconut fatty
  acids                                                 18 g
- oxyethylenated dodecylphenol                          17 g

0097810

- dipropylene glycol                20.5 g

- perfume                      as required.

| European Patent Office | | | | |

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X,Y | DE-A-2 057 957 (ORSYMONDE) * Claims 1,2,7; page 5, lines 24-26; page 21, example 25 * | 1,3-5 | A 61 K 7/08 |
| X,Y | FR-A-2 074 498 (AJINOMOTO) * Claims; page 10, table VII; page 11, lines 5-6 * | 1,5 | |
| X,Y | US-A-3 953 353 (BARRETT et al.) * Claims 1,2,5; column 2, lines 28-39; column 4, line 24 - column 5, line 68; column 6, lines 59-66 * | 1,3-6 | |
| A | US-A-3 519 570 (McCARTHY) * Column 4, lines 46-60 * | 1 | |
| X,Y | DE-A-2 164 993 (UNILEVER) * Claims 1-3,5; page 2, lines 7-30; page 3, line 20 - page 4, line 4; example 1; page 10, table * | 1-6 | TECHNICAL FIELDS SEARCHED (Int. Cl. ³) A 61 K 7/00 C 12 N 9/00 C 11 D 3/00 |
| Y | FR-A-1 579 878 (SPOFA) * Abstract; example 2; page 2, lines 5-14, 34-38 * | 1,4-6 | |

--- -/-

The present search report has been drawn up for all claims

| Place of search | Date of completion of the search | Examiner |
|---|---|---|
| THE HAGUE | 26-09-1983 | WILLEKENS G.E.J. |

## European Patent Office

### DOCUMENTS CONSIDERED TO BE RELEVANT

Page  2

| Category | Citation of document with indication, where appropriate, of relevant passages | Relevant to claim | CLASSIFICATION OF THE APPLICATION (Int. Cl. ³) |
|---|---|---|---|
| Y | AU-A- 37 836 (O. MAYER & CO.)(1972)<br>* Claims; page 1, lines 1-20; page 4, line 1 - page 6, line 5 * | 1,4 | |
| A | DE-B-1 242 794 (SAPHIR)<br>* Examples; claim * | 1,5 | |

TECHNICAL FIELDS SEARCHED (Int. Cl. ³)

The present search report has been drawn up for all claims

| Place of search | Date of completion of the search | Examiner |
|---|---|---|
| THE HAGUE | 26-09-1983 | WILLEKENS G.E.J. |